# EUROPEAN PATENT APPLICATION

(11) **EP 1 033 116 A1**
(43) Date of publication of application: **06.09.2000**
(21) Application number: 00104132.6
(22) Date of filing: 29.02.2000
(51) Int. Cl.: A61F 2/36, A61F 2/30

(54) **Hip prosthesis**

(30) Priority: 03.03.1999 IT UD990048
(71) Applicant: LIMA Lto SpA, 33030 Villanova di S. Daniele (UD) (IT)
(72) Inventor: Lualdi, Gabriele, 33034 Fagagna (UD) (IT)
(74) Representative: Petraz, Gilberto Luigi

(57) **Abstract**

Hip prosthesis to restore articulation to a damaged or deteriorated hip, said prosthesis comprising a cotyloid component, or acetabulum cup, able to be housed inside the acetabulum of the hip, and a femoral component (10), constrained rotatably in the form of a spherical joint to said iliac component and able to be associated with the femur, said femoral component (10) comprising a stem (11), able to be inserted and attached axially to the femur, a neck (12) associated or able to be associated with the upper part of the stem (11) in correspondence with a defined fitting and connection zone (10a), and a head, (13) supported by the neck (12), able to be coupled with said iliac component, said femoral component (10) being lined on the outside, at least in correspondence with said fitting and connection zone (10a), with a netting (15) woven in meshes (17) able to encourage the organic re-growth of the bone tissue of the femur and its anchorage to the walls of said femoral component (10).

## Description

### FIELD OF THE INVENTION

This invention concerns a hip prosthesis as set forth in the main claim.

The invention is used in the field of orthopaedics, and in particular in the field of arthro-prosthetics, in order to restore articulation to a damaged or deteriorated hip, following an illness or an accident.

### BACKGROUND OF THE INVENTION

The state of the art includes prostheses by means of which articulation is restored to a damaged or deteriorated hip.

These prostheses comprise a cotyloid component, called acetabulum cup, able to be associated with the acetabulum, and a femoral component able to be attached inside the femur by removing the neck of the femur itself.

The acetabulum cup is substantially hemispherical in shape and is equipped with a cavity, also substantially hemispherical, defining the seating which joins it with the femoral component.

The femoral component comprises a stem, of an elongated conformation, substantially conical or cylindrical or of another appropriate morphology, able to be inserted and attached inside a longitudinal channel made in the upper part of the femur, called the diaphysis channel.

A substantially cylindrical element, called the neck, is associated with the stem, and is able to support a head with a shape mating with the cavity of the acetabulum cup and which can be inserted therein.

The neck, which is arranged inclined with respect to the stem, may be made in a single piece with the latter or may be an element in itself.

Once the prosthesis has been applied, the femoral component is constrained with the head inside the acetabulum cup and is tree to rotate according to the movements of the articulation of the hip.

During the application of the prosthesis, the acetabulum cup and the stem of the femoral component may be attached, respectively inside the acetabulum and inside the diaphysis channel, according to different techniques, also depending on the type of prosthesis used.

After the operation, the biological regeneration of the bone tissue, which anchors to the walls of the components as it grows, causes the definitive attachment of the prosthesis.

In those cases where the bone tissue is compromised, its regeneration is limited and therefore insufficient to anchor adequately to the walls of the components of the prosthesis, particularly to those of the stem of the femoral component.

The definitive attachment of the implant is therefore in these cases extremely precarious and may cause considerable problems for the patient.

There have been proposals for stems having walls lined with porous material, or with grooves or little cavities, in order to encourage the anchorage of the bone tissue thereon, and therefore determine a more stable attachment of the prosthesis.

These solutions have not always proved satisfactory, and have also entailed an increase in the costs of the stems because it is necessary to make complex supplementary work on the walls thereof.

The present Applicant has devised and embodied this invention to overcome these shortcomings and to obtain other advantages.

### SUMMARY OF THE INVENTION

The invention is set forth and characterized in the main claim, while the dependent claims describe other characteristics of the invention.

The purpose of the invention is to provide a hip prosthesis which is able to be integrated in an optimum manner with the bone tissue of the organism into which it is implanted, ensuring a satisfactory definitive attachment, particularly in correspondence with the stem of the femoral component.

Another purpose of the invention is to provide a hip prosthesis which will not entail the need for particular work during the production step, and can be produced at a reasonable cost.

The prosthesis according to the invention substantially comprises the same elements as hip prostheses of a conventional type and has a femoral component lined on the outside, at least in correspondence with the meta-epiphysis zone of the femur, by a thin netting woven in a mesh.

According to a preferential form of the invention, the netting is made with woven strands of titanium.

In a first embodiment, the strands are arranged orthogonal to each other and define substantially square meshes.

According to a variant, the metal strands are crossed diagonally and define rhomboid meshes.

According to a variant, in correspondence with the zone where the netting is applied, the femoral component has a lower part in the surface, with a depth mating with the thickness of the netting.

The netting applied to the femoral component has characteristics which encourage the regeneration of the bone tissue, allowing it to grow through the mesh of the netting and therefore encouraging it to anchor to the walls of the femoral component.

Therefore, by means of this invention, with an extremely simple and economic solution, we obtain a prosthesis which ensures in a brief time an extremely stable biological attachment of the femoral component inside the diaphysis channel.

### BRIEF DESCRIPTION OF THE DRAWINGS

These and other characteristics of the invention will become clear from the following description of a preferential form of embodiment, given as a non-restrictive example, with reference to the attached drawings wherein:
- Fig. 1: is a front view of the femoral component of the hip prosthesis according to the invention;
- Fig. 2: is a side view of the femoral component in Fig. 1;
- Figs. 3a and 3b: show two possible configurations of the netting used for the hip prosthesis according to the invention;
- Fig. 4: shows a variant of Fig. 1.

### DETAILED DESCRIPTION OF PREFERRED EMBODIMENT

With reference to the attached Figures, number 10 denotes generally the femoral component of the hip prosthesis according to the invention.

The femoral component 10 essentially comprises a stem 11, a neck 12 and a head 13.

The stem 11, which in this case is substantially conical in shape, is able to be partly inserted and attached inside the diaphysis channel of the femur.

The neck 12 is associated inclined to the stem 11, in correspondence with a fitting and connection zone 10a of the middle-upper segment of the femoral component 10, and is able to support the head 13 by means of one of its ends 12a.

The head 13 is hemispherical in shape and is able to be coupled with an acetabulum cup (not shown here) which constitutes the cotyloid component of the hip prosthesis.

According to one characteristic of the invention, the femoral component 10 is lined on the outside, in the fitting and connection zone 10a, with a thin netting 15 woven in a close mesh.

The netting 15 is attached to the femoral component 10 with conventional systems, for example by using rivets, welds or other similar attachment means, and is made of an at least partly flexible material, compatible with the human organism.

In a preferential embodiment of the invention, the netting 15 is made of titanium.

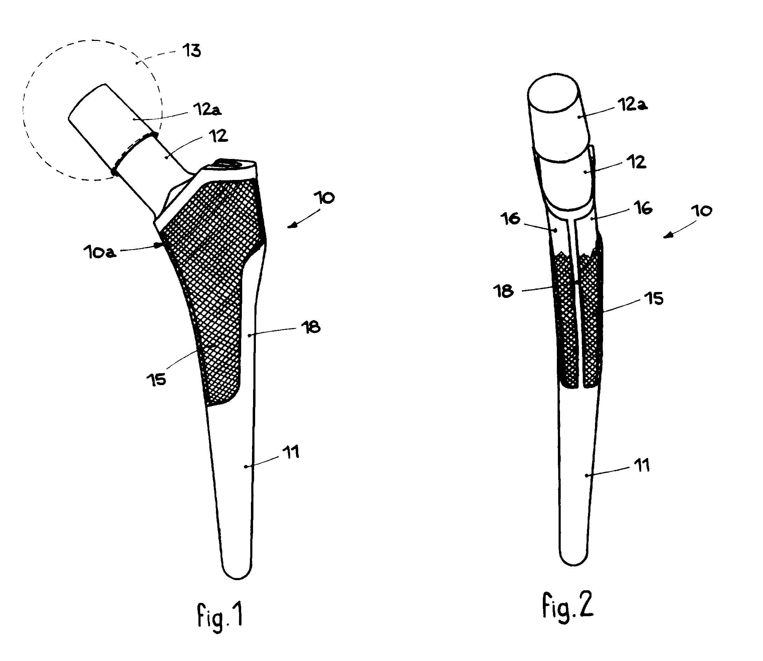

In the solution shown in Figs. 1 and 2, the neck 12 is made in a single piece with the stem 11 and the netting 15 is associated in correspondence with two lower parts 16 in the surface, made in the middle-upper segment of the stem 11.

The depth of the lower parts 16, in this case separated by two longitudinal ribs 18, is advantageously mating with the thickness of the netting 15, so that the latter is substantially contained in the lower parts 16.

According to a variant, the lower parts 16 are less deep than the thickness of the netting 15 which is therefore slightly protruding with respect to the femoral component 10.

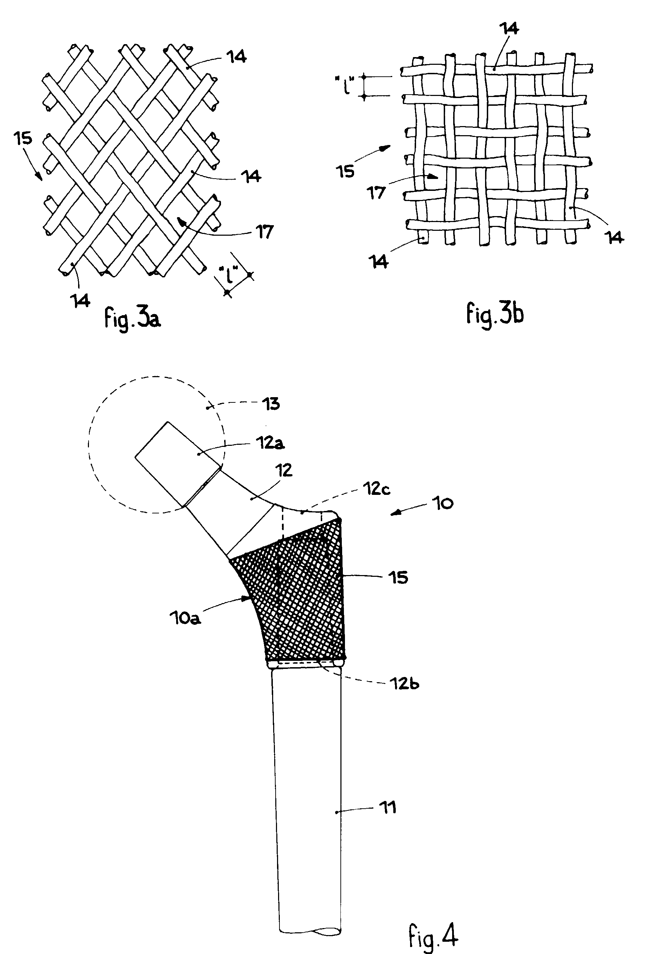

On the contrary, the femoral component 10 shown in Fig. 4 comprises a stem 11 and a neck 12 consisting of autonomous elements associated with each other by inserting and attaching the stem 11 inside an insertion cavity 12b made in the lower part of the neck 12.

In this case, the stem 11 is attached by using appropriate screw means introduced through a hole 12c connected to the insertion cavity 12b.

In the femoral component 10, the fitting and connection zone 10a is substantially defined by the lower part of the neck 12; the netting 15 is therefore attached in correspondence therewith.

Moreover, in this case, the netting 15 is attached directly onto the outer surface of the fitting and connection zone 10a and is advantageously arranged so as to leave the hole 12c free.

The strands 14 which make up the netting 15 applied to the femoral component 10 may be woven with different warps so as to define different configurations of the netting 15.

Figs. 3a and 3b show two preferential warp patterns for the netting 15.

In a first solution shown in Fig. 3a, the strands 14 which make up the netting 15 are woven according to an alternate warp with a diagonal arrangement and define rhomboid meshes 17.

In the embodiment shown in Fig. 3b, the strands 14 are woven orthogonally to each other according to an alternate warp and define substantially square meshes 17.

According to the invention, the meshes 17 have an inner size such as to encourage the growth of the bone tissue through the netting 15, so that the bone tissue can anchor to the walls of the femoral component 10.

In a preferential embodiment, the measurement of the inner side "1" of the meshes 17 is advantageously between 0.3 mm and 1.5 mm.

However, it is obvious that modifications and additions may be made to the hip prosthesis as described heretofore, but these shall remain within the field and scope of the invention.

For example the netting 15 may include meshes 17 of a different conformation or extend even more along the femoral component 10.

There may also be present a single lower part 16 in the surface where the netting 15 is applied, extending continuously around the stem 11 or the neck 12.

## Claims

1. Hip prosthesis to restore articulation to a damaged or deteriorated hip, said prosthesis comprising a cotyloid component, or acetabulum cup, able to be housed inside the acetabulum of the hip, and a femoral component (10), constrained rotatably in the form of a spherical joint to said iliac component and able to be associated with the femur, said femoral component (10) comprising a stem (11), able to be inserted and attached axially to the femur, a neck (12) associated or able to be associated to the upper part of the stem (11) in correspondence with a defined fitting and connection zone (10a), and a head (13), supported by the neck (12), able to be coupled with said iliac component, the prosthesis being characterized in that said femoral component (10) is lined on the outside, at least in correspondence with said fitting and connection zone (10a), with a netting (15) woven in meshes (17) able to encourage the organic re-growth of the bone tissue of the femur and its anchorage to the walls of said femoral component (10).

2. Hip prosthesis as in Claim 1, characterized in that said netting (15) is made of titanium.

3. Hip prosthesis as in Claim 1, characterized in that said netting (15) is attached to the femoral component (10) by means of rivets, welds or other similar means.

4. Hip prosthesis as in Claim 1, characterized in that said netting (15) is attached directly onto the outer surface of said fitting and connection zone (10a).

5. Hip prosthesis as in Claim 1, characterized in that said netting (15) is attached in correspondence with at least a lower part (16) made in the surface of said fitting and connection zone (10a).

6. Hip prosthesis as in Claim 5, characterized in that said netting (15) is attached in correspondence with two or more lower parts (16) made in the surface and separated from each other by longitudinal ribs (18).

7. Hip prosthesis as in Claim 5 or 6, characterized in that the depth of said lower parts (16) is mating with the thickness of said netting (15).

8. Hip prosthesis as in Claim 7, characterized in that the depth of said lower parts (16) is less than the thickness of said netting (15).

9. Hip prosthesis as in Claim 1, characterized in that said netting (15) has meshes (17) of a substantially rhomboid conformation.

10. Hip prosthesis as in Claim 1, characterized in that said netting (15) has meshes (17) of a substantially square conformation.

11. Hip prosthesis as in Claim 1, characterized in that the maximum inner size of said meshes (17) is between 0.3 mm and 1.5 mm.
